# FASCICULE DE BREVET EUROPEEN

(11) **EP 0 470 989 B1**
(45) Date de publication et mention de la délivrance du brevet: **20.07.1994**
(21) Numéro de dépôt: 90907149.0
(22) Date de dépôt: 02.05.1990
(51) Int. Cl.: C12P 21/00

(54) **Utilisation des anticorps monoclonaux anti-B2 microglobuline**
Verwendung von monoklonalen Antikörpern gegen B2-Mikroglobulin
Use of anti-B2 microglobulin monoclonal antibodies

(30) Priorité: 03.05.1989 FR 8905915
(43) Date de publication de la demande: 19.02.1992
(73) Titulaire: INSTITUT NATIONAL DE LA SANTE ET DE LA RECHERCHE MEDICALE (INSERM), 75654 Paris Cédex 13 (FR)
(72) Inventeur: CHERMANN, Jean-Claude, F-13011 Marseille (FR); CORBEAU, Pierre, F-34000 Montpellier (FR); DEVAUX, Christian, F-13001 Marseille (FR); KOURILSKY, François, F-13008 Marseille (FR); REY, Françoise, F-13008 Marseille (FR)
(74) Mandataire: Ahner, Francis
(86) Numéro de dépôt international: FR9000311
(87) Numéro de publication internationale: WO9013657

(56) Documents cités:
- Human Immunology, vol. 18, no. 1, 1987, Elsevier Science Publishing Co., Inc., (New York, NY, US), J.A. Hoxie et al., pp. 39-52
- Biological Abstracts, vol. 81, no. 5, 1986, (Philadelphia, PA, US), S. Gupta, p. AB-474, abstract 43287
- Chemical Abstracts, vol. 112, no. 12, 21 Mqy 1990, (Columbus, Ohio, US), P. Corbeau et al., p. 547, abstract 196502p

## Description

La présente invention se rapporte à l'utilisation des anticorps monoclonaux anti-B₂ microglobuline.

Récemment, il a été mis en évidence par les inventeurs que des anticorps monoclonaux pouvaient intervenir sur la production du virus HIV 1.

Plus précisément, la présente invention se rapporte à l'utilisation d'un anticorps monoclonaux anti-B₂ microglobuline pour l'obtention d'un médicament destiné au traitement ou à la prévention de maladies lieés à une infection à HIV
Il a ainsi été montré que la majorité des anticorps monoclonaux anti-B₂ microglobuline retardaient la formation de sincytia dans les cellules MT4 infectées et retardaient également fortement la production du HIV 1 dans les cellules mononucléaires infectées du sang périphérique.

L'interférence de ces anticorps vis-à-vis de la production dudit virus se réaliserait de préférence lors du cycle de réplication virale ou encore lors de la réplication ou conception de la particule virale.

La présente invention se rapporte également à l'utilisation à titre de médicament destiné au traitement ou à la prévention de maladies liées à une infection virale d'un de ces anticorps de même qu'aux compositions pharmaceutiques contenant à titre de principe actif au moins un anticorps selon l'invention.

De telles compositions pharmaceutiques pourraient contenir egalement d'autres composants actifs thérapeutiquement ou non.

Les exemples et figures données ci-dessous permettront de mettre en évidence d'autres avantages et caractéristiques de la présente invention.

La figure 1 représente l'effet des anticorps monoclonaux anti-B₂ microglobuline, HC11-151-1, B11G6, B2-62-2 ou C21-48A, sur la production de HIV 1 par des cellules mononucléaires du sang périphérique (PBMC). Les PBMC ont été incubées avec ou sans anticorps monoclonaux.

La figure 2 représente l'activité des anticorps monoclonaux anti-B₂ microglobuline B11G6 sur des cellules MT4 infectées par le HIV 1.

La figure 3 représente la production de HIV 1 par des cellules mononucléaires de sang périphérique cultivées et non pré-incubées avec des anticorps monoclonaux anti-B₂ microglobuline B11G6.

### Matériels et méthodes

### 1) Anticorps monoclonaux

Les liquides ascitiques d'anti-B₂ microglobuline, (B2µG) B11G6, B2-62-2 et C21-48A, ont été fournis par la Société Immunotech S.A. (Immunotech, Marseille, France).

Le liquide ascitique d'anti-B₂ microglobuline HC11-151-1 a été produit et caractérisé dans le laboratoire. Les anticorps monoclonaux ont été utilisés aux concentrations suivantes : 100 µg/ml pour les fluides ascitiques du B11G6, 1/50c pour ceux du B2-62-2, C21-48A et HC11-151-1.

### 2) Les cellules

La lignée cellulaire MT4 immortalisée T positive aux HTLV1 CD4 hautement réceptive à l'effet cytopathogénique du HIV 1, dérive de patients et a été fournie par N. YAMAMOTO. Des cellules sont cultivées dans un milieu RPMI 1640 (GIBCO, Paisley, Ecosse) additionné de 10 % de sérum de veau foetal (Bio-Lab Jerusalem Israël), 1 % mélange d'antibiotique PSN et 1 % de glutamine (GIBCO, Paisley, Ecosse).

Les cellules mononucléaires du sang périphérique.

Elles ont été obtenues à partir de donneurs de sang volontaires par leucophérèse. Elles ont été isolées à partir d'une centrifugation à un gradien de densité Ficoll-Hypraque, lavées et stockées à 70°C. Après décongélation, les cellules mononucléaires de sang périphérique ont été cultivées à un taux de 10⁶ cellules par ml dans un milieu de culture de lignée cellulaire additionnée avec IL-2 (10 % de lymphocult-T-LF. Biotest Diagnostic, Allemagne), du sérum anti-interféron (28 µ/ml, MILES, PARIS, FRANCE) et de polylrène (2 µg/ml, SIGMA, St Louis, MO, U.S.A), 3 jours avec de la phytobemagglutine A (pHA 1/1000, DIFCO, Detroit, Michigan) et 4 autres jours sans celui-ci avant l'essai d'infection.

### 3) Essai d'infection au HIV

3.10⁵ cellules MT4 ou 10⁵ cellules PBMC sont incubées ou non durant 1 heure à 4°C dans une plaque à 96 micropuits (COSTAR, Cambridge, MA) avec des anticorps monoclonaux dans un milieu cellulaire de 100 µl. Les cellules sont lavées 3 fois avec 200 µl et exposées 1 heure à 4°C à 100 µl d'HIV 1 (HIV 1 -BRU) (activité réverse transcriptase 1,5.10⁶ cpm/ml) et dilué à 10⁻³ (pour MT4) ou 10⁻¹ (pour PBMC). Les cellules sont lavées ensuite 5 fois puis cultivées avec ou sans anticorps monoclonaux à 37°C dans une plaque à 24 micropuits (COSTAR, Cambridge MA), sous une atmosphère de CO₂ à 5 % en duplicate pour les cellules MT4 et dans des ballons à 25 cm² (COSTAR, Cambridge MA) pour le PBMC. L'infection des cellules MT4 par du VIH 1 a été suivie tous les jours en évaluant la formation de syncitia et en observant l'effet cytopathogénique au microscope optique. L'infection de PBMC a été contrôlée deux fois par semaine en mesurant l'activité de la réverse transcriptase.

### 4) Essai sur l'inhibition de la formation de syncitia

10⁵ de cellules mononucléaires de sang périphérique infectées chroniquement par du HIV 1, lavées 3 fois, ont été mélangées avec 3. 10⁴ de cellules non infectées MT4 dans un milieu de culture de lignée cellulaire (300 µl) avec ou sans anticorps monoclonaux, cultivées toute une nuit, en duplicate 9 heures dans une plaque à micropuits à 37°C, sous une atmosphère de CO₂ à 5 % et la formation de syncitia a été évaluée au microscope optique.

### EXEMPLE 1

### Essai d'anticorps monoclonaux anti B₂ microglobulines sur la production de HIV 1 sur les cellules MT4 et les cellules mononucléaires du sang périphérique

### 1) Les anticorps monoclonaux anti-B₂ microglobulines retardent la production de HIV 1 par les cellules mononucléaires du sang périphérique.

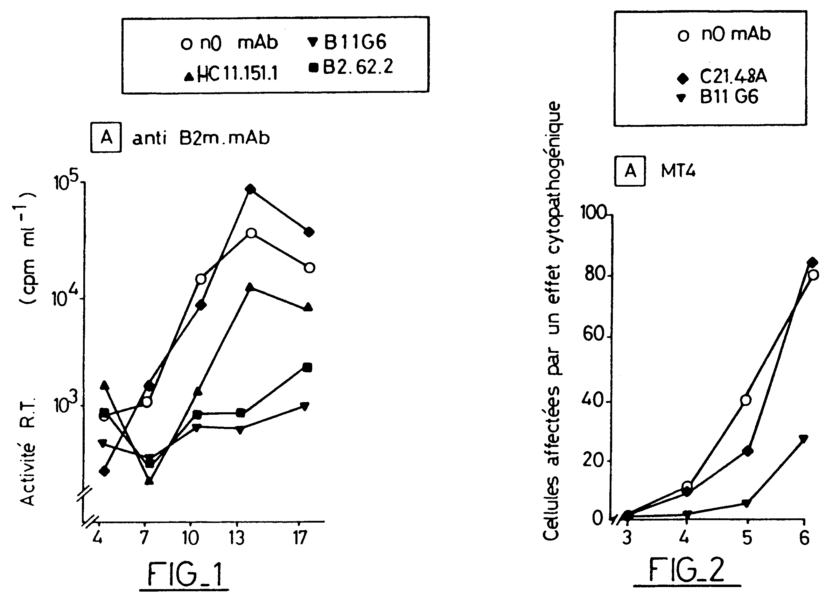

L'effet des anticorps monoclonaux anti-B₂ microglobulines sur la production de HIV 1 par les cellules mononucléaires du sang périphérique incubées avant l'infection et cultivées juste après avec ces anticorps monoclonaux a été évalué en contrôlant l'activité réverse transcriptase de leur surnageant (figure 1).

Les anticorps monoclonaux anti-B₂ microglobulines retardent l'effet cytopathogénique induit par le virus HIV 1 dans les cellules MT4.

Pour déterminer si les anticorps monoclonaux anti-B₂ microglobulines pouvaient retarder également la production de HIV 1 dans les cellules MT4, ces cellules ont été incubées avec des anticorps monoclonaux, infectées avec du HIV 1 puis cultivées avec des anticorps monoclonaux et l'effet cytopathogénique a été évalué quotidiennement (figure 2), le B11G6 mais non le C21-48A retarde l'apparition de l'effet cytopathogénique.

### EXEMPLE 2

### Activité des anticorps monoclonaux anti-B₂ microglobulines B11G6 sur la pénétration du HIV 1 dans les cellules mononucléaires du sang périphérique

Afin de voir si les anticorps monoclonaux anti-B₂ microglobulines pouvaient empêcher la pénétration du HIV 1, les cellules mononucléaires du sang périphérique ont été pré-incubées avec des anticorps monoclonaux, infectées avec du HIV 1 mais cultivées sans anticorps.

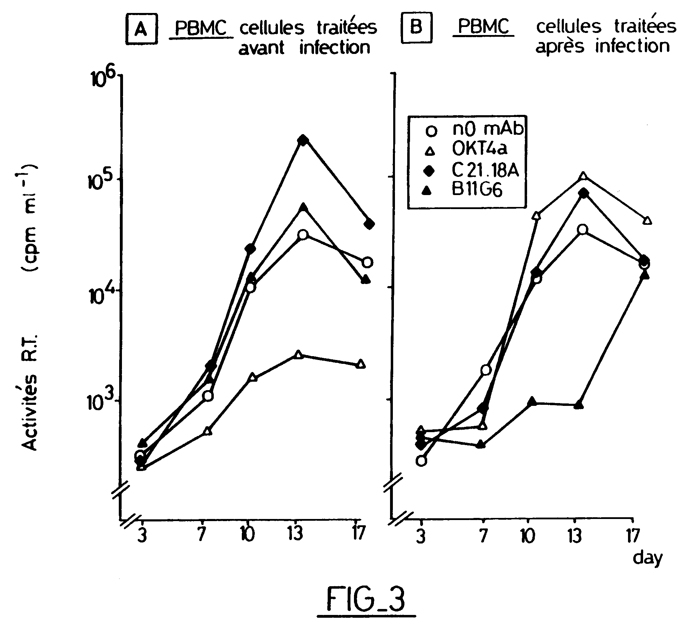

La figure 3 montre que la pré-incubation avec le B11G6 réduit la production du HIV 1 par les cellules mononucléaires du sang périphérique.

### EXEMPLE 3

### Activité des anticorps monoclonaux anti-B₂ microglobulines sur la culture de cellules mononucléaires du sang périphérique infectées par du HIV 1

Dans l'expérience suivante, les cellules mononucléaires du sang périphérique ont été infectées avec du HIV 1, lavées puis immédiatement cultivées avec des anticorps monoclonaux.

La figure 3b montre que la culture avec le B11G6 retarde l'apparition de l'activité de la réverse transcriptase dans le surnageant des cellules mononucléaires du sang périphérique.

## Revendications

1. Utilisation d'un anticorps monoclonal anti-β₂ microglobuline pour l'obtention d'un médicament destiné au traitement ou à la prévention de maladies liées à une infection à HIV.

2. Utilisation selon la revendication 1, dans laquelle on traite les cellules CD4 du corps humain.

3. Utilisation selon l'une des revendications 1 et 2, dans laquelle on traite les cellules mononucléaires de sang périphérique.

4. Utilisation selon l'une des revendications 1 à 3, dans laquelle les anticorps monoclonaux anti-β₂ microglobuline sont choisis parmi : B11-G-6, B2-62-2 et HC11-151-1.

## Claims

1. Use of a monoclonal anti β₂-microglobulin antibody for the preparation of a medicament intended for the treatment or the prevention of diseases linked to a HIV infection.

2. Use according to claim 1, wherein the CD4 cells of human body are treated.

3. Use according to one of claims 1 and 2, wherein the peripheral blood mononuclear cells are treated.

4. Use according to one of claims 1 to 3, wherein the anti β₂-microglobulin monoclonal antibodies are choosen among : B11-G-6, B2-62-2 and HC11-151-1.

## Patentansprüche

1. Verwendung eines monoklonalen anti-β₂-Mikroglobulin-Antikörpers zur Herstellung eines Medikamentes für die Behandlung oder Vorbeugung von Erkrankungen, die mit einer HIV-Infektion verbunden sind.

2. Verwendung nach Anspruch 1, bei der man Zellen CD4 des menschlichen Körpers behandelt.

3. Verwendung nach einem der Ansprüche 1 und 2, bei der man mononukleare Zellen von peripherem Blut behandelt.

4. Verwendung nach einem der Ansprüche 1 bis 3, bei der die monoklonalen anti-β₂-Mikroglobulin-Antikörper ausgewählt werden unter: B11-G-6, B2-62-2 und HC11-151-1.
